# FASCICULE DE BREVET EUROPEEN

(11) **EP 2 074 983 B1**
(45) Date de publication et mention de la délivrance du brevet: **15.03.2017**
(21) Numéro de dépôt: 08171186.3
(22) Date de dépôt: 05.11.2003
(51) Int. Cl.: A61K 8/898, A61Q 5/12, B82Y 5/00, A61K 8/41, A61K 8/06, B01F 17/00

(54) **Procédé de préparation de nanoémulsion cationique et composition cosmétique**
Präparationsverfahren einer kationischen Nanoemulsion und kosmetische Zusammensetzung
Method for preparing a cationic nanoemulsion and cosmetic composition

(30) Priorité: 29.11.2002 FR 0215080
(43) Date de publication de la demande: 01.07.2009
(62) Demande divisionnaire de: 03292766.7
(73) Titulaire: L'Oréal, 75008 Paris (FR)
(72) Inventeur: Dubief, Claude, 78150 Le Chesnay (FR); Fack, Géraldine, 92300 Levallois (FR); Nicolas-Morgantini, Luc, 60810 Rully (FR); Restle, Serge, 95390 Saint-Prix (FR)
(74) Mandataire: Dossmann, Gérard

(56) Documents cités:
- EP-A- 0 820 758
- EP-A- 1 120 102
- EP-A- 1 129 684
- WO-A-01/56537
- DE-A1- 19 710 155
- US-A- 5 298 240
- US-A- 5 468 725

## Description

La présente invention concerne un procédé de préparation d'une nanoémulsion cationique, une composition cosmétique susceptible d'être obtenue par ledit procédé et une utilisation de ladite composition.

Les microémulsions et nanoémulsions de l'art antérieur sont bien connues en cosmétique et sont recherchées pour leurs propriétés cosmétiques. Elles permettent notamment d'obtenir un démêlage, une douceur, un toucher, une rinçabilité et un effet conditionneur des matières kératiniques telles que les cheveux, qui sont meilleures par rapport aux émulsions et dispersions classiques utilisées dans ce domaine.

Les microémulsions et nanoémulsions sont généralement obtenues soit par un procédé d'homogénéisation sous haute pression, soit par un procédé à température d'inversion de phase. Cependant, ces deux procédés présentent des inconvénients majeurs.

En effet, le procédé d'homogénéisation sous haute pression nécessite un appareillage spécifique et particulièrement lourd pour pouvoir travailler sous des pressions importantes allant de 12.10⁷ à 18.10⁷ Pa. Ce procédé n'est donc pas facile à mettre en oeuvre dans l'industrie.

Le procédé à température d'inversion de phase (ou procédé PIT) conduit quant à lui à des nanoémulsions dont leur granulométrie est rarement inférieure à 100 nm.

Les demandes de brevet EP 1 129 684 et EP 1 120 102 décrivent des nanoémulsions huile-dans-eau dont les globules d'huile ont une taille moyenne en nombre inférieure à 150 nm, qui comprennent au moins une huile, au moins un lipide amphiphile de préférence non ionique ou anionique et au moins un polymère cationique ou non ionique comportant au moins une séquence hydrophobe et au moins une séquence hydrophile, avec un rapport pondéral de la quantité d'huile sur la quantité de lipide amphiphile compris entre 1 et 10. Ces nanoémulsions sont préparées par homogénéisation à haute pression.

La Demanderesse a alors découvert de manière surprenante qu'en mélangeant sous agitation dans l'ordre suivant, les composants de la phase grasse et des tensioactifs non ioniques, à une température supérieure aux températures de fusion des composants de la phase grasse et des tensioactifs non ioniques, et sous pression atmosphérique normale, puis de l'eau, suivie de l'ajout d'au moins un tensioactif cationique, on obtenait des nanoémulsions cationiques de type huile-dans-eau, présentant une granulométrie moyenne en nombre inférieure à 100 nm.

Ces nanoémulsions cationiques obtenues avec ce procédé présentent en outre des propriétés cosmétiques telles qu'un démêlage, une douceur, un toucher, et une rinçabilité et un effet conditionneur, qui sont nettement améliorées par rapport aux propriétés cosmétiques des nanoémulsions de l'art antérieur.

Ce procédé est également plus facile à mettre en oeuvre que les deux procédé connus de l'art antérieur cités ci-dessus, et ne nécessitent pas d'appareillage spécifique.

La présente invention a donc pour objet un procédé de préparation de nanoémulsion cationique.

Un autre objet de l'invention consiste en une composition cosmétique sous la forme d'une nanoémulsion cationique susceptible d'être obtenue par ledit procédé.

L'invention a également pour objet une utilisation de ladite composition comme composition de nettoyage, de teinture ou de permanente, comme composition de prétraitement ou de post-traitement des teintures, permanentes, décolorations et défrisages.

L'invention a encore pour objet un procédé de traitement des matières kératiniques mettant en oeuvre ladite composition.

D'autres objets, caractéristiques, aspects et avantages de l'invention apparaîtront encore plus clairement à la lecture de la description et des divers exemples qui suivent.

Selon l'invention, le procédé de préparation de nanoémulsion cationique comprend les étapes suivantes :
(a) mélange sous agitation, d'au moins un composé gras et d'au moins un tensioactif non ionique, de préférence d'au moins deux tensioactifs non ioniques, à une température Tₘ supérieure à la température de fusion du ou des composés gras et du ou des tensioactifs non ioniques, sous pression atmosphérique normale, le diagramme de phase ternaire composé(s) gras/tensioactif(s) non ionique(s)/eau présentant au moins une zone d'existence d'une phase nanoémulsion de type huile-dans-eau et les concentrations du ou des composés gras et du ou des tensioactifs non ioniques étant choisies de telle sorte que l'on puisse atteindre cette zone de nanoémulsion par simple dilution à l'eau,
(b) ajout de l'eau, sous agitation, de manière à atteindre cette zone de nanoémulsion, et
(c) ajout à la nanoémulsion ainsi obtenue, d'au moins un tensioactif cationique.

Le choix des proportions des composants de la nanoémulsion se fait donc à l'aide du diagramme de phase ternaire phase grasse/phase émulsionnante non ionique/eau dans lequel la phase grasse est constituée d'au moins un composé gras tel que décrit ci-dessous, et la phase émulsionnante non ionique est constituée d'au moins un tensioactif non ionique tel que décrit ci-dessous. Ce diagramme permet de déterminer la zone où il y a formation d'une nanoémulsion de type huile-dans-eau. Un tel diagramme est présenté dans la Figure 1 dans laquelle p représente la zone de formation de ladite nanoémulsion.

Une fois le diagramme de phase ternaire établi selon des techniques bien connues de l'homme du métier, on choisit les proportions de la phase émulsionnante et de la phase grasse de telle sorte que le rapport pondéral τ composé(s) gras/tensioactif(s) non ionique(s) soit compris entre A et B, points représentés sur la Figure 1.

Ce rapport pondéral τ est généralement compris entre 0,1 et 1,5 et mieux encore entre 0,1 et 1.

La température Tₘ est de préférence comprise entre la température ambiante et 100 °C, mieux encore entre 20 °C et 85 °C. Par température ambiante, on entend une température de l'ordre de 20 °C.

De préférence, l'addition d'eau se fait aux environs de la température Tₘ, et encore plus préférentiellement à une température Θ comprise entre Tₘ et Tₘ-20°C.

La quantité de composé(s) gras utilisée dans l'étape (a) est généralement comprise entre 1 et 30 % en poids, de préférence entre 1 et 20 % en poids, mieux encore entre 4 et 12 % en poids par rapport au poids total de la nanoémulsion cationique.

La quantité de tensioactif(s) non ionique(s) utilisée dans l'étape (a) est comprise entre 2 et 30 % en poids, de préférence entre 2 et 20 % en poids, mieux encore entre 8 et 20 % en poids par rapport au poids total de la nanoémulsion cationique.

La quantité d'eau généralement ajoutée dans l'étape (b) du procédé est comprise entre 40 et 97 % en poids, de préférence entre 50 et 90 % en poids et plus particulièrement de 65 à 90% en poids par rapport au poids total de la nanoémulsion cationique.

Une fois la nanoémulsion obtenue, on ajoute au moins un tensioactif cationique. Le tensioactif cationique ajouté est de préférence sous forme d'une solution ou d'une dispersion dans l'eau. La température d'addition du tensioactif cationique n'est pas essentielle mais de préférence on la choisira proche de Θ ou de Tₘ.

La quantité de tensioactif(s) cationique(s) utilisée dans l'étape (c) est généralement comprise entre 0,1 et 10 % en poids, de préférence entre 0,2 et 6 % en poids par rapport au poids total de la nanoémulsion cationique.

La phase de refroidissement à température ambiante peut se faire avant ou après l'étape (c). Dans les deux cas, la taille des particules est conservée au cours de ce refroidissement, ce qui n'est notamment pas le cas dans un procédé PIT.

Le procédé selon l'invention permet d'obtenir une nanoémulsion dont les particules présentent une taille moyenne en nombre inférieure à 100 nm, de préférence comprise entre 10 et 100 nm, et encore plus préférentiellement entre 20 et 90 nm.

La taille moyenne en nombre des particules peut être déterminée en particulier selon la méthode connue de diffusion quasi-élastique de la lumière. A titre d'appareil utilisable pour cette détermination, on peut citer l'appareil de marque BROOKHAVEN équipé d'un banc optique SX 200 (avec laser à 532 nm), et d'un corrélateur BI 9000. Cet appareil fournit une mesure du diamètre moyen par spectroscopie de corrélation de photon (ou PCS : "*Photon Correlation Spectroscope*") qui permet de déterminer le diamètre moyen en nombre à partir du facteur de polydispersité également mesuré par l'appareil.

On peut en outre caractériser la nanoémulsion par mesure de sa turbidité selon la méthode NTU à l'aide d'un turbidimètre de modèle 2100P de HACH Company, à température ambiante. La turbidité des nanoémulsions de l'invention est généralement inférieure à 400 unités NTU, et de préférence comprise entre 50 et 250 unités NTU.

Les composés gras utilisables dans le procédé selon l'invention sont de préférence choisis parmi les esters d'acide gras, les huiles végétales transestérifiées ou non, et leurs mélanges.

Comme ester d'acide gras, on peut notamment citer les composés de formule RₐCOOR_{b} dans laquelle Rₐ représente le reste d'un acide gras, saturé ou insaturé, comportant de 6 à 29 atomes de carbone, de préférence de 8 à 22 atomes de carbone, et R_{b} représente une chaîne hydrocarbonée saturée ou insaturée, contenant de 1 à 30 atomes de carbone, de préférence de 1 à 12 atomes de carbone, tels que l'huile de Purcellin (octanoate de stéaryle), le myristate d'isopropyle, le palmitate d'isopropyle, le stéarate de butyle, le laurate d'hexyle, l'adipate de diisopropyle, l'isononanoate d'isononyle, le palmitate de 2-éthylhexyle, le laurate de 2-hexyldécyle, le palmitate de 2-octyldécyle, le myristate ou le lactate de 2-octyldodécyle.

Comme huile végétale, on peut notamment mentionner l'huile d'amande douce, l'huile d'avocat, l'huile de ricin, l'huile d'olive, la cire de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum.

Comme huile végétale transestérifiée, on utilise de préférence l'huile d'olive transestérifiée avec de l'hexanol, la cire de jojoba transestérifiée avec de l'éthanol.

Les composés gras particulièrement préférés dans le cadre de la présente invention sont le myristate d'isopropyle, l'isononanoate d'isononyle, la cire de jojoba, l'huile d'olive transestérifiée avec de l'hexanol, la cire de jojoba transestérifiée avec de l'éthanol, et leurs mélanges.

Les agents tensioactifs non-ioniques utilisables dans le procédé de l'invention sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les huiles végétales hydrogénées ou non, polyoxyalkylénées (2-50 moles d'oxyde d'alkylène), de préférence polyoxyéthylénées ou polyoxypropylénées, les mono-, di- ou triglycérides d'acide gras en C₈₋₃₀, les alcools polyoxyéthylénés et/ou polyoxypropylénés, les alpha-diols polyoxyéthylénés et/ou polyoxypropylénés, les alkylphénols polyoxyéthylénés et/ou polyoxypropylénés, ayant une chaîne grasse comportant par exemple 8 à 18 atomes de carbone, le nombre de groupements oxyde d'éthylène ou oxyde de propylène pouvant aller notamment de 2 à 50, et leurs mélanges. On peut également citer les copolymères d'oxyde d'éthylène et de propylène, les condensats d'oxyde d'éthylène et de propylène sur des alcools gras ; les amides gras polyoxyéthylénés ayant de préférence de 2 à 30 moles d'oxyde d'éthylène, les amides gras polyglycérolés comportant en moyenne 1 à 5 groupements glycérol et en particulier 1,5 à 4 ; les esters d'acides gras du sorbitane ou les esters d'acides gras du sorbitane polyoxyéthylénés ayant de 2 à 30 moles d'oxyde d'éthylène ; les esters d'acides gras du saccharose, les esters d'acides gras, de préférence en C₈₋₃₀, du polyéthylèneglycol, les esters d'acide gras en C₈₋₃₀ du polyglycérol, les alkylpolyglycosides, les dérivés de N-alkylglucamine, les oxydes d'amines tels que les oxydes d'(alkyl en C₁₀-C₁₄)amine ou les oxydes de N-acylaminopropylmorpholine.

Les tensioactifs non ioniques particulièrement préférés dans l'invention sont notamment choisis parmi l'huile de ricin hydrogénée polyoxyéthylénée à 35 moles d'oxyde d'éthylène (désignée ci-après "à 35 OE"), l'huile de ricin hydrogénée polyoxyéthylénée à 7 moles d'oxyde d'éthylène (ou à 7 OE), l'huile d'olive polyoxyéthylénée à 7 OE, les mono-oléates de sorbitane à 4 OE, 5 OE ou à 20 OE, les (alkyl en C12-C14)glycosides ou (alkyl en C8-C14)glycosides, le monostéarate de glycérol à 30 OE, le mono-oléate de décaglycéryle, l'alcool oléïque polyoxyalkyléné à 2 ou 10 OE, l'alcool laurique polyoxyéthyléné à 7 OE, le dioléate de méthylglucoside et leurs mélanges.

Les tensioactifs cationiques pouvant être utilisés dans le procédé selon l'invention, sont ceux bien connus en soi, tels que les sels d'amines grasses primaires, secondaires ou tertiaires, éventuellement polyoxyalkylénées, les sels d'ammonium quaternaire, et leurs mélanges.

A titre de tensioactifs cationiques, on préfère plus particulièrement les sels d'ammonium quaternaires, tels que, par exemple :
- ceux qui présentent la formule générale (I) suivante : 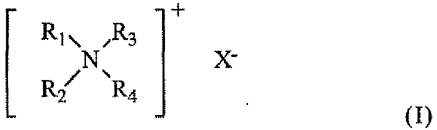 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre et les halogènes. Les radicaux aliphatiques sont, par exemple, choisis parmi les radicaux alkyle, alcoxy, polyoxyalkylène (C₂-C₆), alkylamide, alkyl(C₁₂-C₂₂)amidoalkyle(C₂-C₆), alkyl(C₁₂-C₂₂)acétate, hydroxyalkyle, comportant environ de 1 à 30 atomes de carbone ; X est un anion choisi dans le groupe des halogénures, phosphates, acétates, lactates, alkyl(C₁-C₆)sulfates, (alkyl en C₁-C₆)- ou (alkyl en C₁-C₆)aryl-sulfonates ;
- les sels d'ammonium quaternaire de l'imidazoline, comme par exemple ceux de formule (II) suivante : 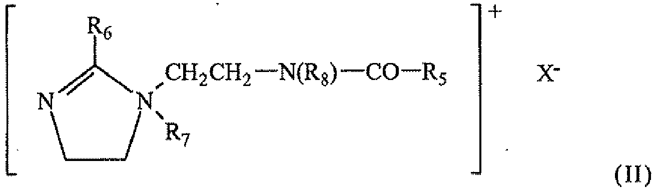 dans laquelle R₅ représente un radical alcényle ou alkyle comportant de 8 à 30 atomes de carbone, par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X⁻ est un anion choisi dans le groupe des halogénures, phosphates, acétates, lactates, alkylsulfates, alkyl- ou alkylaryl-sulfonates. De préférence, R₅ et R₆ désignent un mélange de radicaux alcényle ou alkyle comportant de 12 à 21 atomes de carbone, par exemple dérivés des acides gras du suif, R₇ désigne un radical méthyle, R₈ désigne un atome d'hydrogène. Un tel produit est par exemple commercialisé sous la dénomination REWOQUAT^{®} W 75 par la société REWO ;
- les sels de diammonium quaternaire de formule (III) : 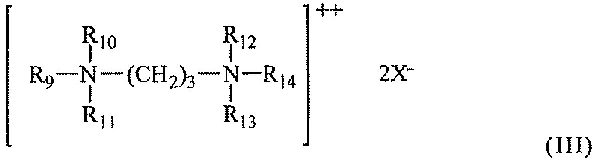 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates.

De tels sels de diammonium quaternaire comprennent notamment le dichlorure de propanesuif diammonium ;
- les sels d'ammonium quaternaire contenant au moins une fonction ester, tels que ceux de formule (IV) suivante : 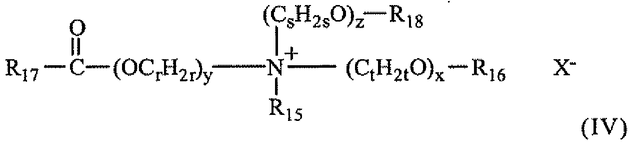 dans laquelle :
   R₁₅ est choisi parmi les radicaux alkyles en C₁-C₆ et les radicaux hydroxyalkyles ou dihydroxyalkyles en C₁-C₆ ;
   R₁₆ est choisi parmi :
      - le radical 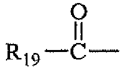
      - les radicaux R₂₀ hydrocarbonés en C₁-C₂₂, linéaires ou ramifiés, saturés ou insaturés,
      - l'atome d'hydrogène,
   R₁₈ est choisi parmi :
      - le radical 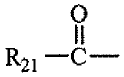
      - les radicaux R₂₂ hydrocarbonés en C₁-C₆, linéaires ou ramifiés, saturés ou insaturés,
      - l'atome d'hydrogène,
   R₁₇, R₁₉ et R₂₁, identiques ou différents, sont choisis parmi les radicaux hydrocarbonés en C₇-C₂₁, linéaires ou ramifiés, saturés ou insaturés ;
   r, s et t, identiques ou différents, sont des entiers valant de 2 à 6 ;
   y est un entier valant de 1 à 10 ;
   x et z, identiques ou différents, sont des entiers valant de 0 à 10 ;
   X⁻ est un anion simple ou complexe, organique ou inorganique ;
   sous réserve que la somme x + y + z vaut de 1 à 15, que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle, et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone, ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, r, s et t, identiques ou différents, valent 2 ou 3, et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement dans la composition selon l'invention, les sels d'ammonium de formule (IV) dans laquelle :
- R₁₅ désigne un radical méthyle ou éthyle,
- x et y sont égaux à 1 ;
- z est égal à 0 ou 1 ;
- r, s et t sont égaux à 2 ;
- R₁₆ est choisi parmi :
   - le radical 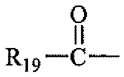
   - les radicaux méthyle, éthyle ou hydrocarbonés en C₁₄-C₂₂,
   - l'atome d'hydrogène ;
- R₁₈ est choisi parmi :
   - le radical 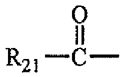
   - l'atome d'hydrogène ;
- R₁₇, R₁₉ et R₂₁, identiques ou différents, sont choisis parmi les radicaux hydrocarbonés en C₁₃-C₁₇, linéaires ou ramifiés, saturés ou insaturés, et de préférence parmi les radicaux alkyles et alcényles en C₁₃-C₁₇, linéaires ou ramifiés, saturés ou insaturés.

Avantageusement, les radicaux hydrocarbonés sont linéaires.

On peut citer par exemple les composés de formule (IV) tels que les sels (chlorure ou méthylsulfate notamment) de diacyloxyéthyl-diméthylammonium, de diacyloxyéthyl-hydroxyéthyl-méthylammonium, de monoacyloxyéthyl-dihydroxyéthyl-méthylammonium, de triacyloxyéthyl-méthylammonium, de monoacyloxyéthyl-hydroxyéthyl-diméthylammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus, par exemple, par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale, ou par transestérification de leurs esters méthyliques. Cette estérification est suivie d'une quaternisation à l'aide d'un agent d'alkylation tel qu'un halogénure d'alkyle (méthyle ou éthyle de préférence), un sulfate de dialkyle (méthyle ou éthyle de préférence), le méthanesulfonate de méthyle, le para-toluènesulfonate de méthyle, la chlorhydrine du glycol ou du glycérol.

De tels composés sont par exemple commercialisés sous les dénominations DEHYQUART^{®} par la société HENKEL, STEPANQUAT^{®} par la société STEPAN, NOXAMIUM^{®} par la société CECA, REWOQUAT^{®} WE 18 par la société REWO-WITCO.

Comme mélange de sels d'ammonium, on peut utiliser par exemple le mélange contenant 15 à 30 % en poids de méthylsulfate d'acyloxyéthyl-dihydroxyéthyl-méthylammonium, 45 à 60% de méthylsulfate de diacyloxyéthyl-hydroxyéthyl-méthylammonium et 15 à 30% de méthylsulfate de triacyloxyéthyl-méthylammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Parmi les sels d'ammonium quaternaire de formule (I), on préfère d'une part, les chlorures de tétraalkylammonium comme, par exemple, les chlorures de dialkyldiméthylammonium ou d'alkyltriméthylammonium dans lesquels le radical alkyle comporte environ de 12 à 22 atomes de carbone, en particulier le chlorure de béhényltriméthylammonium (Genamin^{®} KDMP de Clariant), les chlorures de distéaryldiméthylammonium, de cétyltriméthylammonium, de benzyldiméthyl-stéarylammonium ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans le procédé de l'invention sont choisis parmi les sels d'ammonium quaternaire, et en particulier parmi le chlorure de palmitylamidopropyltriméthylammonium, le chlorure de cétyl-triméthylammonium et le chlorure de béhényl-triméthylammonium.

La présente invention concerne aussi une composition cosmétique, sous la forme d'une nanoémulsion cationique de type huile-dans-eau, dont les particules présentent une taille de moyenne en nombre inférieure à 100 nm, de préférence comprise entre 10 et 100 nm, encore plus préférentiellement entre 20 et 90 nm. Elle comprend au moins un composé gras, au moins 2 à 30% en poids d'un, de préférence au moins deux tensioactifs non ioniques, au moins un tensioactif cationique, et de l'eau en des proportions telles que le rapport pondéral τ composé(s) gras/tensioactif(s) non ionique(s) soit compris entre 0,1 et 1,5, et encore plus préférentiellement entre 0,1 et 1,
le tensioactif cationique étant choisi parmi :
- ceux qui présentent la formule générale (V) suivante : 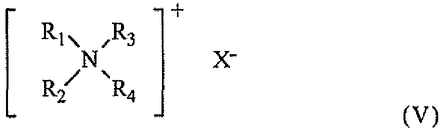 dans laquelle le radical R₁ représente un radical aliphatique, linéaire ou ramifié, comportant de 8 à 30 atomes de carbone, ou un radical aromatique tel que aryle ou alkylaryle, les radicaux R₂, R₃ et R₄, qui peuvent être identiques ou différents, représentent un radical aliphatique, linéaire ou ramifié, comportant de 1 à 6 atomes de carbone, en particulier alkyle ou hydroxyalkyle ; X est un anion choisi dans le groupe des halogénures, phosphates, acétates, lactates, alkyl(C₁-C₆)sulfates, (alkyl en C₁-C₆)- ou (alkyl en C₁-C₆)aryl-sulfonates ;
   Les radicaux R1 aliphatiques peuvent comporter des hétéroatomes tels que notamment l'oxygène, l'azote, le soufre et les halogènes. Les radicaux aliphatiques sont, par exemple, choisis parmi les radicaux alkyle, alcoxy, alkylamide, alkyl(C₁₂-C₂₂)amidoalkyle(C₂-C₆), alkyl(C₁₂-C₂₂)acétate, hydroxyalkyle, comportant environ de 8 à 30 atomes de carbone,
- les sels d'ammonium quaternaire de l'imidazoline, comme par exemple ceux de formule (II) ci-dessus
- les sels de diammonium quaternaire de formule (III) ci-dessus.
- les sels d'ammonium quaternaire contenant au moins une fonction ester, tels que ceux de formule (IV) ci-dessus.

Parmi les sels d'ammonium quaternaire de formule (V), on préfère les chlorures d'alkyltriméthylammonium dans lesquels le radical alkyle comporte environ de 12 à 22 atomes de carbone, en particulier le chlorure de béhényltriméthylammonium (Genamin^{®} KDMP de Clariant), les chlorures de cétyltriméthylammonium, ou encore, d'autre part, le chlorure de palmitylamidopropyltriméthylammonium ou le chlorure de stéaramidopropyldiméthyl-(myristyl acétate)-ammonium commercialisé sous la dénomination CERAPHYL^{®} 70 par la société VAN DYK.

Les tensioactifs cationiques particulièrement préférés dans les compositions de l'invention sont choisis parmi les sels d'ammonium quaternaire, et en particulier parmi le chlorure de palmitylamidopropyltriméthylammonium, le chlorure de cétyl-triméthylammonium et le chlorure de béhényl-triméthylammonium.

Les compositions selon l'invention comprennent le(s) composé(s) gras, le(s) tensioactif(s) non ionique(s) et le(s) tensioactif(s) cationique(s) en un rapport pondéral composé(s) gras/(tensioactif(s) non ionique(s) + tensioactif(s) cationique(s)) généralement inférieur à 1,5, de préférence compris entre 0,1 et 1.

Ces compositions sont susceptibles d'être obtenues par le procédé de l'invention tel que décrit ci-dessus.

Le pH des compositions de l'invention est généralement compris entre 3 et 8, de préférence entre 4 et 7.

Les compositions selon l'invention peuvent également contenir des additifs tels que des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des épaississants non polymériques comme des électrolytes, des sucres, des nacrants, des opacifiants, des filtres solaires, des vitamines ou provitamines, des parfums, des colorants, des particules organiques ou minérales, des conservateurs, des agents de stabilisation du pH, des sucres.

L'homme du métier veillera à choisir les éventuels additifs et leur quantité de manière à ce qu'ils ne nuisent pas aux propriétés des compositions de la présente invention.

Ces additifs sont présents dans la composition selon l'invention en une quantité allant de 0 à 50 % en poids par rapport au poids total de la composition.

Les compositions peuvent être utilisées, par exemple, comme composition de nettoyage, de teinture ou de permanente, comme composition de prétraitement ou de post-traitement des shampooings, teintures, permanentes, décolorations et défrisages. De manière préférée, les compositions de l'invention sont des après-shampoings rincés ou non rincés.

La présente invention concerne également un procédé de traitement cosmétique des matières kératiniques qui consiste à appliquer une quantité efficace d'une composition cosmétique telle que décrite ci-dessus, sur les matières kératiniques, à effectuer un éventuel rinçage après un éventuel temps de pose.

Les exemples suivants illustrent la présente invention et ne doivent être considérés en aucune manière comme limitant l'invention.

### EXEMPLES

Les compositions des exemples 1 à 14, selon l'invention, sont préparées selon le mode opératoire de l'invention à partir des ingrédients indiqués dans les tableaux 1 à 3 ci-dessous. In fine, on a obtenu des nanoémulsions cationiques.

**Tableau 1**

| | Quantités en % en poids | | | | |
|---|---|---|---|---|---|
| | Ex. 1 | Ex. 2 | Ex. 3 | Ex. 4 | Ex. 5 |
| Huile de ricin hydrogénée à 35 OE⁽¹⁾ | 9 | 7,5 | 15 | 11,8 | 9,75 |
| Huile de ricin hydrogénée à 7 OE⁽²⁾ | 3 | 2,5 | - | 3,9 | 3,25 |
| Huile d'olive à 7 OE⁽³⁾ | - | - | 5 | - | - |
| Myristate d'isopropyle | 8 | - | - | - | - |
| Isononanoate d'isononyle | - | - | - | - | 7 |
| Cire de jojoba transestérifiée avec de l'éthanol | - | 10 | - | 6,8 | - |
| Cire de jojoba | - | - | - | 1,2 | - |
| Huile d'olive transestérifiée avec de l'hexanol | - | - | 8,6 | - | - |
| Chlorure de palmitylamidopropyl-triméthylammonium⁽⁴⁾ | - | - | - | - | 1,5 |
| Chlorure de cétyltriméthylammonium (MA) | - | - | 2 | - | - |
| Chlorure de béhényl-triméthylammonium⁽⁵⁾ (MA) | 1,6 | 1,6 | - | 1,4 | - |
| Microémulsion de silicone aminée (MA) | - | 1,2 | - | - | - |
| Glycérine | - | - | - | 5 | - |
| Eau qsp | 100 | 100 | 100 | 100 | 100 |

| | | | | | |
|---|---|---|---|---|---|
| MA : Matière active ⁽¹⁾ : vendue sous la dénomination ARLATONE^{®} 980 par la société ICI ⁽²⁾ : vendue sous la dénomination ARLATONE^{®} 989 par la société ICI ⁽³⁾ : vendue sous la dénomination OLIVEM^{®} 300 par la société B&T ⁽⁴⁾ : vendu sous le nom commercial Varisoft^{®} PATC par la société WITCO ⁽⁵⁾ : vendu sous le nom commercial Genamin ^{®} KDMP par la société Clariant GmbH | | | | | |

**Tableau 2**

| | Quantités en % en poids | | | | |
|---|---|---|---|---|---|
| | Ex. 6 | Ex. 7 | Ex. 8 | Ex. 9 | Ex. 10 |
| Mono-oléate de sorbitane à 20 OE⁽¹⁾ | 9,6 | - | - | - | 2,4 |
| Mono-laurate de sorbitane⁽²⁾ | 2,4 | - | - | - | - |
| Alcool oléylique polyoxyéthylénée (10 OE)⁽³⁾ | - | 10 | 9,6 | - | - |
| Alcool oléylique polyoxyéthylénée (2 OE)⁽⁴⁾ | - | - | 2,4 | - | - |
| Alcool en C12-C14 linéaire polyoxyéthylénée (7 OE)⁽⁵⁾ | - | - | - | 12 | - |
| Dioléate de méthylglucoside⁽⁶⁾ | - | - | - | - | 9,6 |
| Myristate d'isopropyle | 8 | 10 | - | 7 | - |
| Isononanoate d'isononyle | - | - | - | - | 8 |
| Cire de jojoba transestérifiée avec de l'éthanol | - | - | 8 | 1 | - |
| Chlorure de cétyltriméthylammonium (MA) | - | - | 2 | 2 | - |
| Chlorure de béhényl-triméthylammonium⁽⁷⁾ (MA) | 1,6 | 1,6 | - | - | 1,5 |
| Microémulsion de silicone aminée (MA) | - | 1,2 | - | - | - |
| Eau qsp | 100 | 100 | 100 | 100 | 100 |

| | | | | | |
|---|---|---|---|---|---|
| ⁽¹⁾ : vendu sous le nom commercial Tween^{®} 80 par la société Uniquema. ⁽²⁾ : vendu sous le nom commercial Span^{®} 20 par la société Uniquema. ⁽³⁾ : vendu sous le nom commercial Brij^{®} 96 par la société Uniquema. ⁽⁴⁾ : vendu sous le nom commercial e Brij^{®} 92 par la société Uniquema. ⁽⁵⁾ : vendu sous le nom commercial Synperonic^{®} L7 par la société Uniquema. ⁽⁶⁾ : vendu sous le nom commercial Glucate^{®} DO par la société Amerchol. ⁽⁷⁾ : vendu sous le nom commercial Genamin^{®} KDMP par la société Clariant GmbH. | | | | | |

**Tableau 3**

| | Quantités en % en poids | | | |
|---|---|---|---|---|
| | Ex. 11 | Ex. 12 | Ex. 13 | Ex. 14 |
| Monostéarate de glycérol (30 OE)⁽¹⁾ | 3,6 | - | - | - |
| Mono-oléate de décaglycéryle⁽²⁾ | 8,4 | 5 | - | - |
| Mono-oléate de sorbitane à 20 OE⁽³⁾ | - | 5 | - | - |
| (Alkyl en C12-C14)glycoside⁽⁴⁾ | - | - | 1,6 | - |
| Mono-laurate de sorbitane à 4 OE⁽⁵⁾ | - | - | 6,4 | - |
| (Alkyl en C8-C14)glycoside⁽⁶⁾ | - | - | - | 3 |
| Mono-oléate de sorbitane à 5 OE⁽⁷⁾ | - | - | - | 7 |
| Mvristate d'isopropyle | 7,5 | - | 12 | 10 |
| Cire de jojoba transestérifiée avec de l'éthanol | - | 10 | - | - |
| Cire de jojoba | 0,5 | - | - | - |
| Chlorure de palmitylamidopropyltriméthylammonium⁽⁸⁾ | - | - | - | 2 |
| Chlorure de cétyltriméthylammonium | - | - | 2 | 2 |
| Chlorure de (béhényl-triméthyl)ammonium⁽⁹⁾ (MA) | 1,6 | 1,6 | - | - |
| Microémulsion de silicone aminée (MA) | - | 1,2 | - | - |
| Glycérine | - | - | - | 5 |
| Eau qsp | 100 | 100 | 100 | 100 |

| | | | | |
|---|---|---|---|---|
| ⁽¹⁾ : vendu sous le nom commercial Tagat^{®} S par la société Goldschmidt. ⁽²⁾ : vendu sous le nom commercial Decaglyn^{®} 1-0 par la société Nikkol. ⁽³⁾ : vendu sous le nom commercial Tween^{®} 80 par la société Uniquema. ⁽⁴⁾ : vendu sous le nom commercial Glucopon^{®} 600 C sup par la société Cognis. ⁽⁵⁾ : vendu sous le nom commercial Tween^{®} 21 par la société Uniquema. ⁽⁶⁾ : vendu sous le nom commercial Glucopon^{®} 650 EC/hh sup par la société Cognis. ⁽¹⁾ : vendu sous le nom commercial Tween^{®} 81 par la société Uniquema. ⁽⁸⁾ : vendu sous le nom commercial Varisoft^{®} PATC par la société WITCO. ⁽⁹⁾ : vendu sous le nom commercial Genamin ^{®} KDMP par la société Clariant GmbH. | | | | |

La taille de particules moyenne en nombre et la turbidité des compositions des exemples 1 à 14, ont été mesurées avec les méthodes telles que décrites plus haut. On a obtenu :
- une taille des particules moyenne en nombre strictement inférieure à 100 nm, et
- une turbidité strictement inférieure à 150 unités NTU.

Appliquées sur cheveux en tant qu'après-shampoings, ces compositions confèrent auxdits cheveux, douceur, souplesse et lissage, tonicité.

## Revendications

1. Composition cosmétique sous forme de nanoémulsion cationique dont les particules présentent une taille moyenne en nombre inférieure à 100 nm, comprenant au moins un composé gras et 2 à 30% en poids d'au moins un tensioactif non ionique en un rapport pondéral 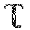 composé(s) gras/tensioactif(s) non ionique(s) compris entre 0,1 et 1,5, au moins un tensioactif cationique et de l'eau,
le tensioactif cationique étant choisi parmi :
- ceux qui présentent la formule générale (I) suivante : 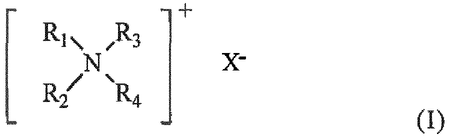 dans laquelle le radical R₁ représente un radical aliphatique, linéaire ou ramifié, comportant de 8 à 30 atomes de carbone, ou un radical aromatique tel que aryle ou alkylaryle, les radicaux R₂, R₃ et R₄, qui peuvent être identiques ou différents, représentent un radical aliphatique, linéaire ou ramifié, comportant de 1 à 6 atomes de carbone ; X est un anion choisi dans le groupe des halogénures, phosphates, acétates, lactates, alkyl(C₁-C₆)sulfates, (alkyl en C₁-C₆)- ou (alkyl en C₁-C₆)aryl-sulfonates ;
- les sels d'ammonium quaternaire de l'imidazoline ;
- les sels de diammonium quaternaire de formule (III) : 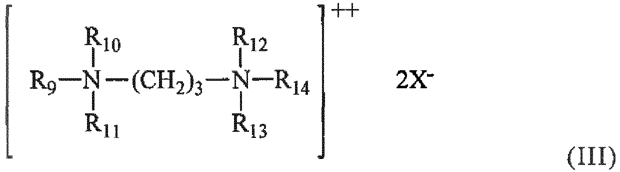 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates ;
- les sels d'ammonium quaternaire contenant au moins une fonction ester.

2. Composition cosmétique selon la revendication 1, caractérisée ce que le rapport pondéral 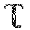 composé(s) gras/tensioactif(s) non ionique(s) est compris entre 0,1 et 1.

3. Composition selon l'une quelconque des revendications précédentes, **caractérisée en ce qu'**elle comprend au moins deux tensioactifs non ioniques.

4. Composition selon l'une quelconque des revendications précédentes, **caractérisée en ce que** le rapport pondéral composé(s) gras/(tensioactif(s) non ionique(s) + tensioactif(s) cationique(s)) est inférieur à 1,5, de préférence compris entre 0,1 et 1

5. Composition cosmétique selon l'une quelconque des revendications précédentes, **caractérisée en ce qu'**elle comprend des additifs choisis parmi des polymères cationiques, anioniques, non ioniques ou amphotères, des silicones non volatiles, modifiées ou non, des épaississants polymériques naturels ou synthétiques, anioniques, amphotères, zwittérioniques, non ioniques ou cationiques, associatifs ou non, des épaississants non polymériques comme des électrolytes, des sucres, des nacrants, des opacifiants, des filtres solaires, des vitamines et provitamines, des parfums, des colorants, des particules organiques ou minérales, des conservateurs, des agents de stabilisation du pH, des sucres.

6. Procédé de préparation de la nanoémulsion cationique selon l'une quelconque des revendications 1 à 5, **caractérisé en ce qu'**il comprend les étapes suivantes :
(a) mélange sous agitation, d'au moins un composé gras et d'au moins un tensioactif non ionique à une température Tₘ supérieure à la température de fusion du ou des composés gras et du ou des tensioactifs non ioniques, sous pression atmosphérique normale, le diagramme de phase ternaire composé(s) gras/tensioactif(s) non ionique(s)/eau présentant au moins une zone d'existence d'une phase nanoémulsion de type huile-dans-eau et les concentrations du ou des composés gras et du ou des tensioactifs non ioniques étant choisies de telle sorte que l'on puisse atteindre cette zone de nanoémulsion par simple dilution à l'eau,
(b) ajout de l'eau sous agitation, de manière à atteindre cette zone de nanoémulsion, et
(c) ajout à la nanoémulsion ainsi obtenue, d'au moins un tensioactif cationique.

7. Procédé selon la revendication 6, **caractérisé en ce que** Tm est comprise entre la température ambiante et 100 °C.

8. Procédé selon la revendication 6 ou 7, **caractérisé en ce que** le rapport pondéral 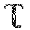 composé(s) gras/tensioactif(s) non ionique(s) est compris entre 0,1 et 1.

9. Procédé selon l'une quelconque des revendications 6 à 8, **caractérisé en ce qu'**il comprend en outre une étape de refroidissement à température ambiante se situant avant ou après l'étape (c).

10. Procédé selon l'une quelconque des revendications 6 à 9, **caractérisé en ce que** le tensioactif cationique est ajouté sous forme d'une solution ou d'une dispersion dans l'eau.

11. Procédé selon l'une quelconque des revendications 6 à 10, **caractérisé en ce que** le composé gras est choisi parmi les esters d'acide gras, les huiles végétales transestérifiées ou non, et leurs mélanges.

12. Procédé selon la revendication 11, **caractérisé en ce que** le composé gras est choisi parmi les composés de formule RₐCOOR_{b} dans laquelle Rₐ représente le reste d'un acide gras supérieur, saturé ou insaturé, comportant de 6 à 29 atomes de carbone, de préférence de 8 à 22 atomes de carbone, et R_{b} représente une chaîne hydrocarbonée, saturée ou insaturée, contenant de 1 à 30 atomes de carbone, de préférence de 1 à 12 atomes de carbone ; l'huile d'amande douce, l'huile d'avocat, l'huile de ricin, l'huile d'olive, la cire de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum ; l'huile d'olive transestérifiée avec de l'hexanol, la cire de jojoba transestérifiée avec de l'éthanol ; et leurs mélanges.

13. Procédé selon la revendication 12, **caractérisé en ce que** le composé gras est choisi parmi le myristate d'isopropyle, l'isononanoate d'isononyle, la cire de jojoba, l'huile d'olive transestérifiée avec de l'hexanol, la cire de jojoba transestérifiée avec de l'éthanol, et leurs mélanges.

14. Procédé selon l'une quelconque des revendications 6 à 13, **caractérisé en ce que** l'on utilise au moins deux tensioactifs non ioniques dans l'étape (a).

15. Procédé selon l'une quelconque des revendications 6 à 14, **caractérisé en ce que** le tensioactif non ionique est choisi parmi les huiles végétales polyoxyalkylénées (2-50 moles d'oxyde d'alkylène), hydrogénées ou non, les mono-, di- ou triglycérides d'acide gras en C₈₋₃₀, les alcools polyoxyéthylénés et/ou polyoxypropylénés, les alpha-diols polyoxyéthylénés et/ou polyoxypropylénés, les alkylphénols polyoxyéthylénés et/ou polyoxypropylénés, ayant une chaîne grasse, le nombre de groupements oxyde d'éthylène ou oxyde de propylène pouvant aller notamment de 2 à 50, les copolymères d'oxyde d'éthylène et de propylène, les condensats d'oxyde d'éthylène et de propylène sur des alcools gras ; les amides gras polyoxyéthylénés ayant de 2 à 30 moles d'oxyde d'éthylène, les amides gras polyglycérolés comportant en moyenne 1 à 5 groupements glycérol et en particulier 1,5 à 4 ; les esters d'acides gras du sorbitane ou les esters d'acides gras du sorbitane polyoxyéthylénés ayant de 2 à 30 moles d'oxyde d'éthylène ; les esters d'acides gras du saccharose, les esters d'acides gras du polyéthylèneglycol, les esters d'acide gras en C₈₋₃₀ du polyglycérol, les alkylpolyglycosides, les dérivés de N-alkyl-glucamine, les oxydes d'amines tels que les oxydes d'(alkyl en C₁₀-C₁₄)amine ou les oxydes de N-acylaminopropylmorpholine et leurs mélanges.

16. Procédé selon la revendication 15, **caractérisé en ce que** le tensioactif non ionique est choisi parmi l'huile de ricin hydrogénée polyoxyéthylénée à 35 OE, l'huile de ricin hydrogénée polyoxyéthylénée à 7 OE, l'huile d'olive polyoxyéthylénée à 7 OE, les mono-oléates de sorbitane à 4 OE, 5 OE ou à 20 OE, les (alkyl en C12-C14)glycosides, ou (alkyl en C8-C14)glycosides, le monostéarate de glycérol à 30 OE, le mono-oléate de décaglycéryle, l'alcool oléïque polyoxyéthylénéeà 2 ou 10 OE, l'alcool laurique polyoxyéthyléné à 7 OE, le dioléate de méthylglucoside et leurs mélanges.

17. Procédé selon la revendication 6, **caractérisé en ce que** les tensioactifs cationiques sont choisis parmi le chlorure de palmitamidopropyltrimonium, le chlorure de cétyl-triméthylammonium et le chlorure de béhényl-triméthylammonium.

18. Procédé selon l'une quelconque des revendications 6 à 17, **caractérisé en ce que** la quantité de composé(s) gras est comprise entre 1 et 30 % en poids, de préférence entre 1 et 20 % en poids par rapport au poids total de la nanoémulsion cationique.

19. Procédé selon l'une quelconque des revendications 6 à 18, **caractérisé en ce que** la quantité de tensioactif(s) non ionique(s) est comprise entre 2 et 20 % en poids par rapport au poids total de la nanoémulsion cationique.

20. Procédé selon l'une quelconque des revendications 6 à 19, **caractérisé en ce que** la quantité d'eau est comprise entre 40 et 97 % en poids, de préférence entre 50 et 90 % en poids par rapport au poids total de la nanoémulsion cationique.

21. Procédé selon l'une quelconque des revendications 6 à 20, **caractérisé en ce que** la quantité de tensioactif(s) cationique(s) est comprise entre 0,1 et 10 % en poids, de préférence entre 0,2 et 6 % en poids par rapport au poids total de la nanoémulsion cationique.

22. Utilisation de la composition cosmétique selon l'une quelconque des revendications 1 à 5, comme composition de nettoyage, de teinture ou de permanente, comme composition de prétraitement ou de post-traitement des shampooings, teintures, permanentes, décolorations et défrisages.

23. Utilisation selon la revendication 22, en tant qu'après-shampooing.

## Patentansprüche

1. Kosmetische Zusammensetzung in Form einer kationischen Nanoemulsion, deren Teilchen eine zahlenmittlere Größe von weniger als 100 nm aufweisen, umfassend mindestens eine Fettverbindung und 2 bis 30 Gew.-% mindestens eines nichtionischen Tensids in einem Gewichtsverhältnis 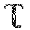 von Fettverbindung bzw. Fettverbindungen zu nichtionischem Tensid bzw. nichtionischen Tensiden zwischen 0,1 und 1,5, mindestens ein kationisches Tensid und Wasser, wobei das kationische Tensid ausgewählt ist aus:
- denjenigen der folgenden allgemeinen Formel (I): 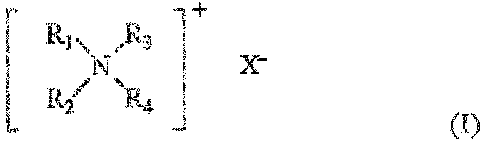 worin der Rest R₁ für einen linearen oder verzweigten aliphatischen Rest mit 8 bis 30 Kohlenstoffatomen oder einen aromatischen Rest wie Aryl oder Alkylaryl steht, die Reste R₂, R₃ und R₄, die gleich oder verschieden sein können, für einen linearen oder verzweigten aliphatischen Rest mit 1 bis 6 Kohlenstoffatomen stehen; X für ein Anion steht, das aus der Gruppe der Halogenide, Phosphate, Acetate, Lactate, (C₁₋₆)-Alkylsulfate, (C₁₋₆)-Alkyl- oder (C₁₋₆)-Alkylarylsulfonate ausgewählt ist;
- quaternären Ammoniumsalzen von Imidazolin;
- quaternären Diammoniumsalzen der Formel (III): 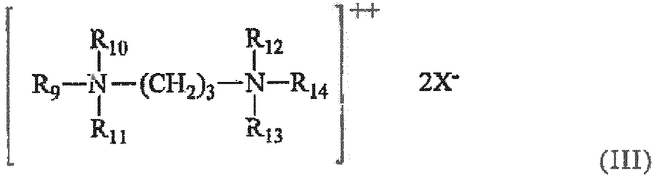
worin R₉ für einen aliphatischen Rest mit ungefähr 16 bis 30 Kohlenstoffatomen steht, R₁₀, R₁₁, R₁₂, R₁₃ und R₁₄, die gleich oder verschieden sind, aus Wasserstoff oder einem Alkylrest mit 1 bis 4 Kohlenstoffatomen ausgewählt sind und X für ein Anion aus der Gruppe der Halogenide, Acetate, Phosphate, Nitrate und Methylsulfate steht;
- quaternären Ammoniumsalzen mit mindestens einer Esterfunktion.

2. Kosmetische Zusammensetzung nach Anspruch 1, **dadurch gekennzeichnet, dass** das Gewichtsverhältnis T von Fettverbindung bzw. Fettverbindungen zu nichtionischem Tensid bzw. nichtionischen Tensiden zwischen 0,1 und 1 liegt.

3. Zusammensetzung nach einem der vorhergehenden Ansprüche, **dadurch gekennzeichnet, dass** sie mindestens zwei nichtionische Tenside umfasst.

4. Zusammensetzung nach einem der vorhergehenden Ansprüche, **dadurch gekennzeichnet, dass** das Gewichtsverhältnis von Fettverbindung bzw. Fettverbindungen zu (nichtionischem Tensid bzw. nichtionischen Tensiden + kationischem Tensid bzw. kationischen Tensiden) unter 1,5 und vorzugsweise zwischen 0,1 und 1 liegt.

5. Kosmetische Zusammensetzung nach einem der vorhergehenden Ansprüche, **dadurch gekennzeichnet, dass** sie Additive umfasst, die aus kationischen, anionischen, nichtionischen oder amphoteren Polymeren, gegebenenfalls modifizierten nichtflüchtigen Silikonen, natürlichen oder synthetischen, anionischen, amphoteren, zwitterionischen, nichtionischen oder kationischen, gegebenenfalls assoziativen polymeren Verdickungsmitteln, nichtpolymeren Verdickungsmitteln wie Elektrolyten, Zuckern, Perlglanzmitteln, Trübungsmitteln, Sonnenschutzmitteln, Vitaminen und Provitaminen, Parfümen, Farbmitteln, organischen oder anorganischen Teilchen, Konservierungsmitteln, Mitteln zur Stabilisierung des pH-Werts und Zuckern ausgewählt sind.

6. Verfahren zur Herstellung der kationischen Nanoemulsion nach einem der Ansprüche 1 bis 5, **dadurch gekennzeichnet, dass** es folgende Schritte umfasst:
(a) Mischen mindestens einer Fettverbindung und mindestens eines nichtionischen Tensids unter Rühren bei einer Temperatur Tₘ oberhalb der Schmelztemperatur der Fettverbindung bzw. der Fettverbindungen und des nichtionischen Tensids bzw. der nichtionischen Tenside unter normalem Atmosphärendruck, wobei das ternäre Phasendiagramm von Fettverbindung bzw. Fettverbindungen, nichtionischem Tensid bzw. nichtionischen Tensiden und Wasser mindestens einen Bereich aufweist, in dem eine Nanoemulsionsphase vom Öl-in-Wasser-Typ vorliegt und wobei die Konzentrationen der Fettverbindung bzw. Fettverbindungen und des nichtionischen Tensids bzw. der nichtionischen Tenside so gewählt sind, dass dieser Nanoemulsionsbereich durch einfaches Verdünnen mit Wasser erreicht werden kann,
(b) Zugeben von Wasser unter Rühren derart, dass dieser Nanoemulsionsbereich erreicht wird, und
(c) Zugeben mindestens eines kationischen Tensids zu der so erhaltenen Nanoemulsion.

7. Verfahren nach Anspruch 6, **dadurch gekennzeichnet, dass** Tₘ zwischen Raumtemperatur und 100°C liegt.

8. Verfahren nach Anspruch 6 oder 7, **dadurch gekennzeichnet, dass** das Gewichtsverhältnis T von Fett-verbindung bzw. Fettverbindungen zu nichtionischem Tensid bzw. nichtionischen Tensiden zwischen 0,1 und 1 liegt.

9. Verfahren nach einem der Ansprüche 6 bis 8, **dadurch gekennzeichnet, dass** es ferner einen Schritt des Abkühlens auf Umgebungstemperatur vor oder nach Schritt (c) umfasst.

10. Verfahren nach einem der Ansprüche 6 bis 9, **dadurch gekennzeichnet, dass** das kationische Tensid in der Form einer Lösung oder einer Dispersion in Wasser zugegeben wird.

11. Verfahren nach einem der Ansprüche 6 bis 10, **dadurch gekennzeichnet, dass** die Fettverbindung aus Fettsäureestern, gegebenenfalls umgeesterten pflanzlichen Ölen und Mischungen davon ausgewählt wird.

12. Verfahren nach Anspruch 11, **dadurch gekennzeichnet, dass** die Fettverbindung aus den Verbindungen der Formel RₐCOOR_{b}, worin Rₐ für den Rest einer gesättigten oder ungesättigten höheren Fettsäure mit 6 bis 29 Kohlenstoffatomen und vorzugsweise 8 bis 22 Kohlenstoffatomen steht und R_{b} für eine gesättigte oder ungesättigte Kohlenwasserstoffkette mit 1 bis 30 Kohlenstoffatomen und vorzugsweise 1 bis 12 Kohlenstoffatomen steht; Süßmandelöl, Avocadoöl, Ricinusöl, Olivenöl, Jojobawachs, Sonnenblumenöl, Getreidekeimöl, Sesamöl, Erdnussöl, Traubenkernöl, Sojaöl, Rapsöl, Distelöl, Kopraöl, Maisöl, Haselnussöl, Sheabutter, Palmöl, Aprikosenkernöl, Calophyllumöl; mit Hexanol umgeestertem Olivenöl, mit Ethanol umgeestertem Jojobawachs und Mischungen davon ausgewählt wird.

13. Verfahren nach Anspruch 12, **dadurch gekennzeichnet, dass** die Fettverbindung aus Isopropylmyristat, Isononylisononanoat, Jojobawachs, mit Hexanol umgeestertem Olivenöl, mit Ethanol umgeestertem Jojobawachs und Mischungen davon ausgewählt wird.

14. Verfahren nach einem der Ansprüche 6 bis 13, **dadurch gekennzeichnet, dass** in Schritt (a) mindestens zwei nichtionische Tenside verwendet werden.

15. Verfahren nach einem der Ansprüche 6 bis 14, **dadurch gekennzeichnet, dass** das nichtionische Tensid aus gegebenenfalls hydrierten polyoxyalkylenierten (2-50 mol Alkylenoxid) pflanzlichen Ölen, C₈-₃₀-Fettsäuremonoglyceriden, -diglyceriden oder -triglyceriden, polyoxyethylenierten und/ oder polyoxypropylenierten Alkoholen, polyoxyethylenierten und/oder polyoxypropylenierten alpha-Diolen, polyoxyethylenierten und/oder polyoxypropylenierten Alkylphenolen, die eine Fettkette aufweisen, wobei die Zahl der Ethylenoxid- bzw. Propylenoxidgruppen im Bereich von 2 bis 50 liegen kann, Copolymeren von Ethylenoxid und Propylenoxid, Kondensaten von Ethylenoxid und Propylenoxid mit Fettalkoholen; polyoxyethylenierten Fettamiden mit 2 bis 30 mol Ethylenoxid, polyglycerinierten Fettamiden, die im Mittel 1 bis 5 Glyceringruppen und insbesondere 1,5 bis 4 Glyceringruppen enthalten; Fettsäureestern von Sorbitan oder polyoxyethylenierten Fettsäureestern von Sorbitan mit 2 bis 30 mol Ethylenoxid; Saccharosefettsäureestern, Polyethylenglykolfettsäureestern; C₈₋₃₀-Fettsäureestern von Polyglycerin, Alkylpolyglycosiden, N-Alkylglucaminderivaten, Aminoxiden, wie (C₁₀₋₁₄)-Alkyl-aminoxiden oder N-Acylaminopropylmorpholinoxiden und Mischungen davon ausgewählt wird.

16. Verfahren nach Anspruch 15, **dadurch gekennzeichnet, dass** das nichtionische Tensid aus mit 35 EO polyoxyethyleniertem hydriertem Ricinusöl, mit 7 EO polyoxyethyleniertem hydriertem Ricinusöl, mit 7 EO polyoxyethyleniertem Olivenöl, Sorbitanmonooleaten mit 4 EO, 5 EO oder 20 EO, (C₁₂₋₁₄)-Alkylglycosiden oder (C₈₋₁₄)-Alkylglycosiden, Glycerylmonostearat mit 30 EO, Decaglycerylmonooleat, mit 2 oder 10 EO polyoxyethyleniertem Oleylalkohol, mit 7 EO polyoxyethyleniertem Laurylalkohol, Methylglucosiddioleat und Mischungen davon ausgewählt wird.

17. Verfahren nach Anspruch 6, **dadurch gekennzeichnet, dass** die kationischen Tenside aus Palmitamidopropyltrimoniumchlorid, Cetyltrimethylammoniumchlorid und Behenyltrimethylammoniumchlorid ausgewählt werden.

18. Verfahren nach einem der Ansprüche 6 bis 17, **dadurch gekennzeichnet, dass** die Menge an Fettverbindung bzw. Fettverbindungen zwischen 1 und 30 Gew.-% und vorzugsweise zwischen 1 und 20 Gew.-%, bezogen auf das Gesamtgewicht der kationischen Nanoemulsion, liegt.

19. Verfahren nach einem der Ansprüche 6 bis 18, **dadurch gekennzeichnet, dass** die Menge an nichtionischem Tensid bzw. nichtionischen Tensiden zwischen 2 und 20 Gew.-%, bezogen auf das Gesamtgewicht der kationischen Nanoemulsion, liegt.

20. Verfahren nach einem der Ansprüche 6 bis 19, **dadurch gekennzeichnet, dass** die Wassermenge zwischen 40 und 97 Gew.-% und vorzugsweise zwischen 50 und 90 Gew.-%, bezogen auf das Gesamtgewicht der kationischen Nanoemulsion, liegt.

21. Verfahren nach einem der Ansprüche 6 bis 20, **dadurch gekennzeichnet, dass** die Menge an kationischem Tensid bzw. kationischem Tensiden zwischen 0,1 und 10 Gew.-% und vorzugsweise zwischen 0,2 und 6 Gew.-%, bezogen auf das Gesamtgewicht der kationischen Nanoemulsion, liegt.

22. Verwendung der kosmetischen Zusammensetzung nach einem der Ansprüche 1 bis 5 als Reinigungszusammensetzung, Färbezusammensetzung oder Dauerwellzusammensetzung, als Zusammensetzung zur Vorbehandlung oder Nachbehandlung von Schamponierungen, Färbungen, Dauerwellen, Bleichungen und Entkräuselungen.

23. Verwendung nach Anspruch 22 als Haarspülung.

## Claims

1. Cosmetic composition in the form of a cationic nanoemulsion, the particles of which exhibit a number-average size of less than 100 nm, comprising at least one fatty compound and from 2 to 30% by weight of at least one nonionic surfactant in a fatty compound(s)/nonionic surfactant(s) ratio by weight τ of between 0.1 and 1.5, at least one cationic surfactant and water,
the cationic surfactant being chosen from:
- those which exhibit the following general formula (I): 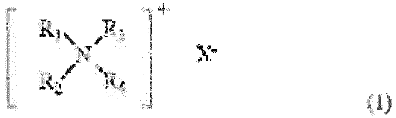 in which the R₁ radical represents a linear or branched aliphatic radical comprising from 8 to 30 carbon atoms or an aromatic radical, such as an aryl or alkylaryl radical, the R₂, R₃ and R₄ radicals, which can be identical or different, represent a linear or branched aliphatic radical comprising from 1 to 6 carbon atoms and X⁻ is an anion chosen from the group of halides, phosphates, acetates, lactates, (C₁-C₆)alkyl sulfates, (C₁-C₆ alkyl)sulfonates or (C₁-C₆ alkyl)arylsulfonates;
- quaternary ammonium salts of imidazolium;
- quaternary diammonium salts of formula (III): 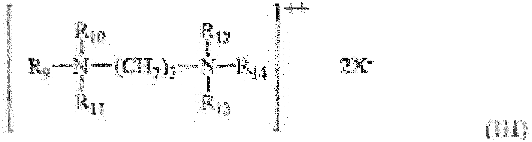 in which R₉ denotes an aliphatic radical comprising approximately from 16 to 30 carbon atoms, R₁₀, R₁₁, R₁₂, R₁₃ and R₁₄, which are identical or different, are chosen from hydrogen or an alkyl radical comprising from 1 to 4 carbon atoms and X⁻ is an anion chosen from the group of the halides, acetates, phosphates, nitrates and methyl sulfates;
- quaternary ammonium salts containing at least one ester functional group.

2. Cosmetic composition according to Claim 1, **characterized in that** the fatty compound(s)/nonionic surfactant(s) ratio by weight τ is between 0.1 and 1.

3. Composition according to either one of the preceding claims, **characterized in that** it comprises at least two nonionic surfactants.

4. Composition according to any one of the preceding claims, **characterized in that** the fatty compound(s)/(nonionic surfactant(s) + cationic surfactant(s)) ratio by weight is less than 1.5, preferably between 0.1 and 1.

5. Cosmetic composition according to any one of the preceding claims, **characterized in that** it comprises additives chosen from cationic, anionic, nonionic or amphoteric polymers, modified or unmodified nonvolatile silicones, natural or synthetic, associative or nonassociative, anionic, amphoteric, zwitterionic, nonionic or cationic polymeric thickeners, nonpolymeric thickeners, such as electrolytes, sugars, pearlescent agents, opacifying agents, sunscreens, vitamins and provitamins, fragrances, colourants, organic or inorganic particles, preservatives or pH-stabilizing agents.

6. Process for the preparation of the cationic nanoemulsion according to any one of Claims 1 to 5, **characterized in that** it comprises the following stages:
(a) mixing, with stirring, of at least one fatty compound and of at least one nonionic surfactant at a temperature Tₘ greater than the melting point of the fatty compound or compounds and of the nonionic surfactant or surfactants, at standard atmospheric pressure, the fatty compound(s)/nonionic surfactant(s)/water ternary phase diagram exhibiting at least one region of existence of a nanoemulsion phase of oil-in-water type and the concentrations of the fatty compound or compounds and of the nonionic surfactant or surfactants being chosen so that it is possible to reach this nanoemulsion region by simple dilution with water,
(b) adding water with stirring, so as to reach this nanoemulsion region, and
(c) adding, to the nanoemulsion thus obtained, at least one cationic surfactant.

7. Process according to Claim 6, **characterized in that** Tₘ is between ambient temperature and 100°C.

8. Process according to Claim 6 or 7, **characterized in that** the fatty compound(s)/nonionic surfactant(s) ratio by weight τ is between 0.1 and 1.

9. Process according to any one of Claims 6 to 8, **characterized in that** it additionally comprises a stage of cooling to ambient temperature located before or after stage (c).

10. Process according to any one of Claims 6 to 9, **characterized in that** the cationic surfactant is added in the form of a solution or of a dispersion in water.

11. Process according to any one of Claims 6 to 10, **characterized in that** the fatty compound is chosen from fatty acid esters, transesterified or nontransesterified vegetable oils and their mixtures.

12. Process according to Claim 11, **characterized in that** the fatty compound is chosen from compounds of formula RₐCOOR_{b} in which Rₐ represents the residue of a saturated or unsaturated higher fatty acid comprising from 6 to 29 carbon atoms, preferably 8 to 22 carbon atoms, and R_{b} represents a saturated or unsaturated hydrocarbon chain containing from 1 to 30 carbon atoms, preferably from 1 to 12 carbon atoms; sweet almond oil, avocado oil, castor oil, olive oil, jojoba wax, sunflower oil, wheat germ oil, sesame oil, peanut oil, grape seed oil, soybean oil, rapeseed oil, safflower oil, coconut oil, maize oil, hazelnut oil, shea butter, palm oil, apricot kernel oil or calophyllum oil; olive oil transesterified with hexanol or jojoba oil transesterified with ethanol; and their mixtures.

13. Process according to Claim 12, **characterized in that** the fatty compound is chosen from isopropyl myristate, isononyl isononanoate, jojoba wax, olive oil transesterified with hexanol, jojoba wax transesterified with ethanol and their mixtures.

14. Process according to any one of Claims 6 to 13, **characterized in that** at least two nonionic surfactants are used in stage (a).

15. Process according to any one of Claims 6 to 14, **characterized in that** the nonionic surfactant is chosen from hydrogenated or nonhydrogenated polyoxyalkylenated (2-50 mol of alkylene oxide) vegetable oils, C₈₋₃₀ fatty acid mono-, di- or triglycerides, polyoxyethylenated and/or polyoxypropylenated alcohols, polyoxyethylenated and/or polyoxypropylenated α-diols, polyoxyethylenated and/or polyoxypropylenated alkylphenols, having a fatty chain, it being possible for the number of ethylene oxide or propylene oxide groups to range in particular from 2 to 50, ethylene oxide and propylene oxide copolymers, condensates of ethylene oxide and propylene oxide with fatty alcohols, polyoxyethylenated fatty amides having from 2 to 30 mol of ethylene oxide, polyglycerolated fatty amides comprising on average from 1 to 5 glycerol groups and in particular from 1.5 to 4, sorbitan fatty acid esters or polyoxyethylenated sorbitan fatty acid esters having from 2 to 30 mol of ethylene oxide, sucrose fatty acid esters, polyethylene glycol fatty acid esters, polyglycerol C₈₋₃₀ fatty acid esters, alcohol polyglycosides, N-alkylglucamine derivatives, amine oxides, such as (C₁₀-C₁₄ alkyl)amine oxides or N-acylaminopropylmorpholine oxides, and their mixtures.

16. Process according to Claim 15, **characterized in that** the nonionic surfactant is chosen from polyoxyethylenated hydrogenated castor oil having 35 EO units, polyoxyethylenated hydrogenated castor oil having 7 EO units, polyoxyethylenated olive oil having 7 EO units, sorbitan mono-oleates having 4 EO, 5 EO or 20 EO units, (C₁₂-C₁₄ alkyl) glycosides or (C₈-C₁₄ alkyl) glycosides, glycerol monostearate having 30 EO units, decaglyceryl mono-oleate, polyoxyethylenated oleyl alcohol having 2 or 10 EO units, polyoxyethylenated lauryl alcohol having 7 EO units, methyl glucoside dioleates and their mixtures.

17. Process according to Claim 6, **characterized in that** the cationic surfactants are chosen from palmitamidopropyltrimonium chloride, cetyltrimethylammonium chloride and behenyltrimethylammonium chloride.

18. Process according to any one of Claims 6 to 17, **characterized in that** the amount of fatty compound(s) is between 1 and 30% by weight and preferably between 1 and 20% by weight, with respect to the total weight of cationic nanoemulsion.

19. Process according to any one of Claims 6 to 18, **characterized in that** the amount of nonionic surfactant(s) is between 2 and 20% by weight, with respect to the total weight of the cationic nanoemulsion.

20. Process according to any one of Claims 6 to 19, **characterized in that** the amount of water is between 40 and 97% by weight, preferably between 50 and 90% by weight, with respect to the total weight of the cationic nanoemulsion.

21. Process according to any one of Claims 6 to 20, **characterized in that** the amount of cationic surfactant(s) is between 0.1 and 10% by weight, preferably between 0.2 and 6% by weight, with respect to the total weight of the cationic nanoemulsion.

22. Use of the cosmetic composition according to any one of Claims 1 to 5 as cleaning, dyeing or permanent wave composition, as composition for the pretreatment or post-treatment of shampooing, dyeing, permanent wave, bleaching and hair straightening operations.

23. Use according to Claim 22, as conditioner.
